# EUROPEAN PATENT SPECIFICATION

(11) **EP 2 641 537 B1**
(45) Date of publication and mention of the grant of the patent: **26.08.2015**
(21) Application number: 12425057.2
(22) Date of filing: 20.03.2012
(51) Int. Cl.: A61B 5/097

(54) **Auxiliary device for collection and sampling of exhaled air**
Hilfsvorrichtung zur Sammlung und Probenahme von Atemluft
Dispositif auxiliaire pour la collecte et l'échantillonnage de l'air expiré

(43) Date of publication of application: 25.09.2013
(73) Proprietor: Universita' Campus Bio-Medico di Roma, 00128 Rome (IT)
(72) Inventor: Pennazza, Giorgio, 00174 Roma (IT); Santonico, Marco, 00173 Roma (IT); Antonelli Incalzi, Raffaele Franco, 00165 Roma (IT); D'Amico, Arnaldo, 00178 Roma (IT); Petriaggi, Massimo, 06049 Spoleto, PG (IT)
(74) Representative: Papa, Elisabetta

(56) References cited:
- EP-A1- 1 096 245
- US-A- 3 661 528
- US-A1- 2004 162 500
- RIEDEL K ET AL: "Determination of benzene and alkylated benzenes in ambient and exhaled air by microwave desorption coupled with gas chromatography-mass spectrometry", JOURNAL OF CHROMATOGRAPHY, ELSEVIER SCIENCE PUBLISHERS B.V, NL, vol. 719, no. 2, 8 January 1996 (1996-01-08), pages 383-389, XP004038456, ISSN: 0021-9673, DOI: 10.1016/0021-9673(95)00737-7

## Description

### Technical Field of the Invention

The present invention refers to an auxiliary device for collection of exhaled air on a sampling means, in particular on an adsorbent cartridge.

### Background

The procedure of collection and analysis of exhaled air from a subject, said air commonly referred to simply as "exhalate" (or "expirate"), can occur directly or indirectly. In the first case, air exhaled by the subject is conveyed to the measuring system without any intermediate step. In the second case, the exhalate sample is stored on an adequate medium in order to be analyzed afterwards.

The indirect procedure is by far the most used one, as the times of the sampling step are generally different from those of the measuring step, making an on-line measuring hardly feasible (with the exception of the measuring of physical parameters such as volume or flow, as it occurs with instruments like the spirometer). Moreover, when a gas chromatograph is used the indirect method becomes the sole one applicable, due to the complexity of the instrument and of the measuring procedure.

There are several exhalate collection media for carrying out the indirect procedure: bags of Tedlar or other low-emission plastic materials, glass vials, stainless steel containers and cartridges containing adsorbent substances (also called *"sorbent traps*").

The media most commonly used are Tedlar bags and cartridges of absorbent material.

Adsorbent cartridges are thin tubes, made of stainless steel or glass, containing a resin or a mixture of adsorbent resins able to trap the volatile organic compounds (VOCs) present in the gas sample which touches them. Adsorbent cartridge operation revolves around the concept of *"breakthrough volume",* or retention volume, as illustrated in *"*Calculation and use of breakthrough volume data", J. J. Manura, Scientific Instrument Services, Inc.

It is defined as volume of carrier gas, per mass unit of adsorbent resin, needed to have the molecules of an analyte travel over the entire length of the tube section containing the resin, and depends on temperature. Retention volume is generally expressed in liters/gram and, considered analyte being equal, it decreases with temperature. This property is fundamental for VOCs trapping: at room temperature, retention volume is sufficiently high to prevent analyte outletting from cartridge during collection, whereas at a high temperature (up to 300°C) retention volume is greatly reduced, allowing the near-immediate release of trapped compounds and therefore allowing their actual sampling over a time deferred with respect to the collection time. Said release procedure is called thermal desorption, therefore adsorbent cartridges are also referred to as "thermal desorption tubes".

Various adsorbent resins are available on the market: the selection is made on the basis of numerous chemico-physical parameters, among which the most relevant ones are retention volumes of analytes of interest at the sampling temperature, and resin affinity for water.

Exhalate collection on cartridges of adsorbent materials offers numerous advantages over bag use. In fact, the mode based on adsorbent cartridges enables to pre-concentrate the sample, thereby enhancing the instrument ability to detect substances at very low concentrations (typically of the order of magnitude of parts per billion, ppb) and guarantees high sample storage stability and great transport practicality, thanks to the reduced dimensions of the cartridges (generally consisting in tubes of a diameter of about 6 mm and a length not exceeding about 20 cm).

However, while bag filling can be carried out without problems by the subject, adsorbent cartridges offer a high resistance to air transit. Because of this, feeding on the cartridges may be carried out directly only by using a pump device pushing exhaled air into the cartridge, which device acts immediately downstream of the patient's mouthpiece. Said methodology is rather complex and requires just the use of a rather complicated pump mechanism, to the detriment of the reliability of the collection and sampling apparatus overall, and therefore of the associated procedure.

In the literature, some devices for exhalate collection on adsorbent cartridge according to the hereto-disclosed mode are described. All use an electric pump for the sampling. In *"*Method for the collection and assay of volatile organic compounds in breath", M. Phillips, Analytical Biochemistry, 1997, 247:272-278, a long heated tube of stainless steel is used, at the proximal end (near to the mouthpiece for the patient) of which lies the inlet for the adsorbent cartridge, followed by a flow sensor and the above-mentioned pump.

In *"*An adaptive breath sampler for use with human subjects with an impaired respiratory function", M. Basanta, T. Koimtzis et al., Analyst 2007, 132:153-163, and in "Construction and evaluation of a versatile CO2 controlled breath collection device", W. Miekisch, A. Hengstenberg et al., IEEE Sensors Journal 2010, 10:211-215, collection and sampling devices are described which, in spite of a large number of functions, have however a very sophisticated (numerous electro-mechanical and electronic components) and not particularly compact configuration.

Other exhaled air collection and sampling devices according to the state of the art can be for example found In US 3.661,528, EP 1096245A1, US 2004/0162500A1 and Riedel et al: "Determination of benzene and alkylated benzenes In ambient and exhaled air by microwave desorption coupled with gas chromatography-mass spectrometry", Journal of Chromatography, vol. 719, no. 2, 8. January 1996, pages 383-389.

Alternatively, exhalate feeding on the cartridges may be carried out indirectly, and in that case the subject fills a bag, whose content is subsequently conveyed into the cartridge by a syringe or an electric pump. This latter method is the most widespread because it does not require the use of specific apparatuses, but entails numerous intermediate steps (in fact,

it Is a "doubly" indirect method) and risks to cause the loss of part of the information contained in exhalate samples, owing to subsequent dilutions and possible contaminations.

Therefore, the known art does not offer a satisfactory solution to the problem of exhalate collection on adsorbent cartridge. In particular, to adsorb the exhalate on the cartridge obtaining an end result of quality and without intermediate steps, only instruments - and therefore procedures - of high complexity are available.

### Summary of the Invention

Therefore, the technical problem set and solved by the present invention is that of providing a device for collection of exhalate on a sampling means, in particular on an adsorbent cartridge, allowing to overcome the drawbacks mentioned above with reference to the known art.

Such a problem is solved by a device according to claim 1.

Preferred features of the present invention are set forth in the dependent claims thereof.

The present invention provides some relevant advantages.

First of all, thanks to its double-chamber structure the device of the invention allows a continuous sampling of exhaled air, even during an inhalation step. The device of the invention allows collection of exhaled air on an adsorbent cartridge in a non-invasive manner. The resistance which the patient perceives during exhalation and inhalation through the device is minimal, guaranteeing a comfortable procedure, also suitable for subjects with breathing difficulties.

The device further allows a high reproducibility of the exhalate sampling procedure.

Moreover, the device is susceptible of a portable and compact embodiment, it being very versatile in clinical practice, of simple use both for the physician and the patient, and apt to be used also on bedridden subjects.

Operation of the device is based on simple principles and the number of components can be limited, so as to guarantee high reliability and reasonable manufacturing and operating costs.

The structural simplicity of the device also fosters full safety of use, without risk of infection for the patient, as it is possible to implement the device so that all parts that may contact the subject be single-use or sterilizable by existing methods.

Other advantages, features and the operation modes of the present invention will be made apparent from the following detailed description of some embodiments thereof, given by way of example and not for limitative purposes.

### Brief Description of the Figures

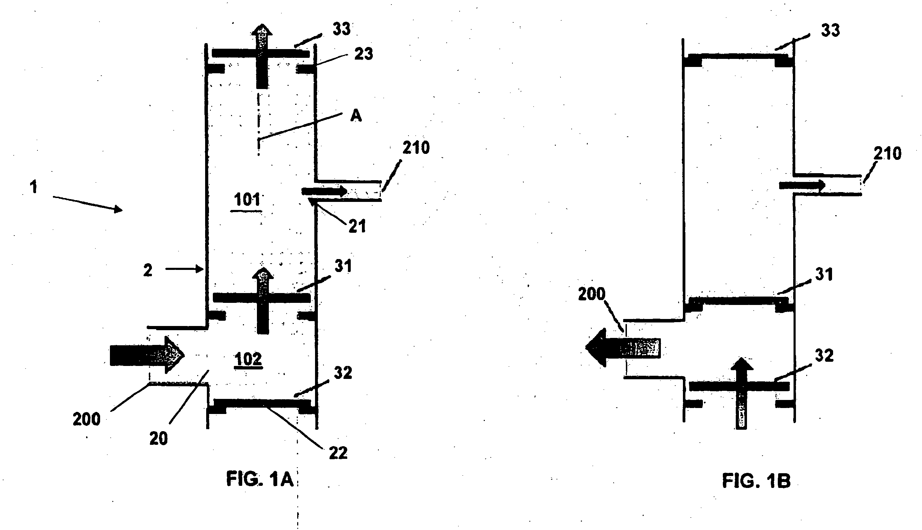

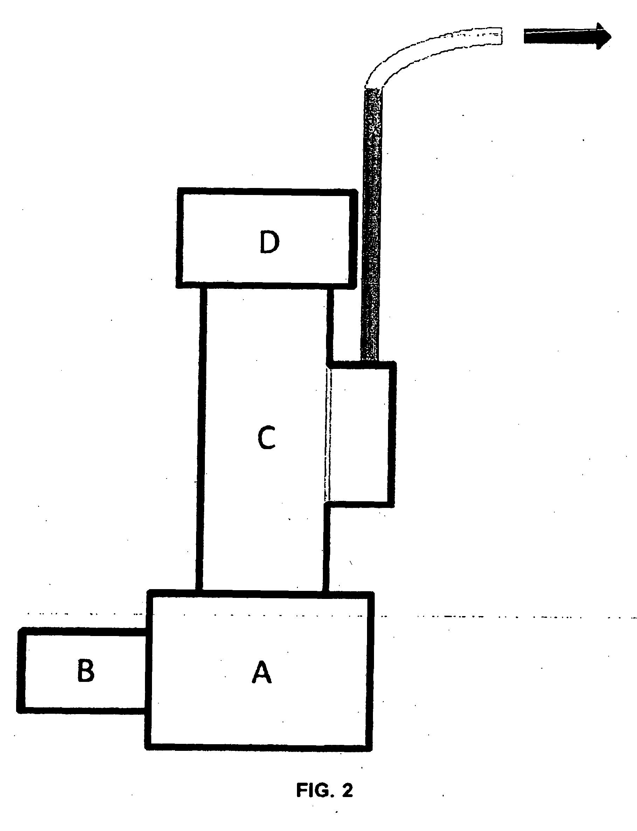

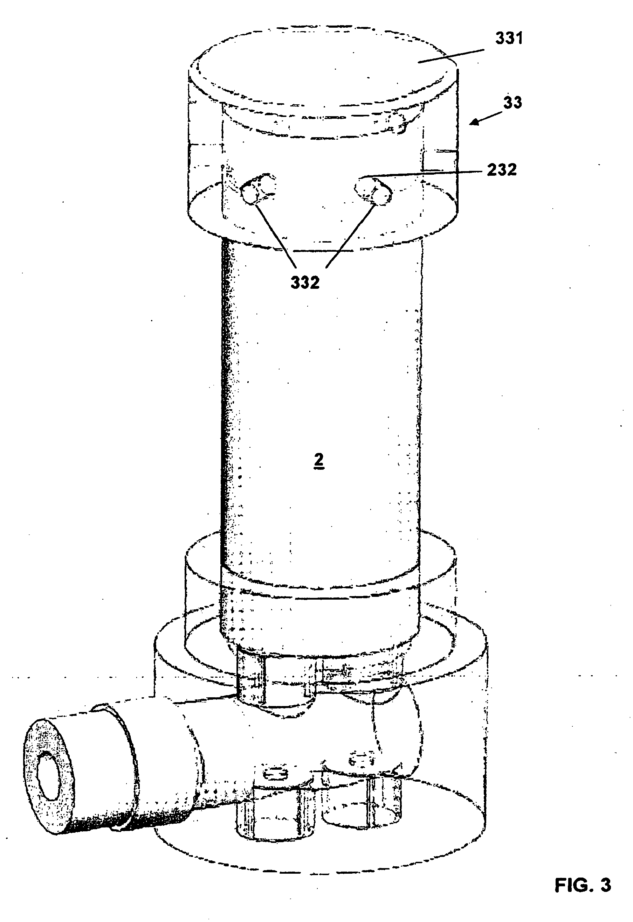

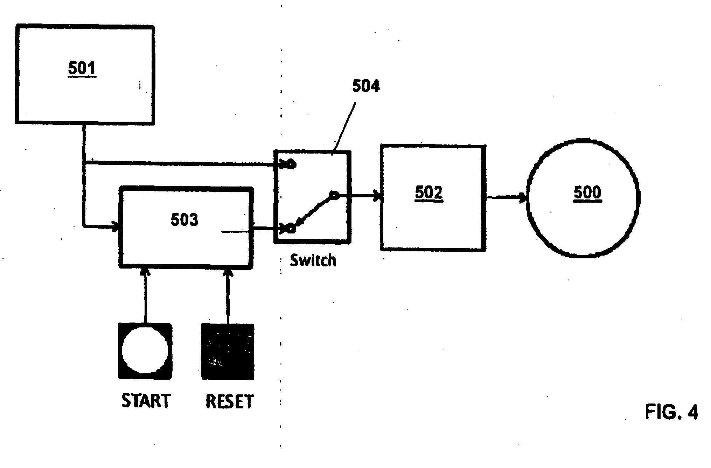

Reference will be made to the figures of the annexed drawings, wherein:
▪ Figures 1A and 1B show each a schematic longitudinal section view of a preferred embodiment of the device of the invention, in a configuration corresponding to exhalation and inhalation, respectively;
▪ Figure 2 shows a schematic side view of the device of Figure 1A, highlighting the operating blocks which implement it;
▪ Figure 3 shows a schematic perspective view, in partial transparency, of the device of Figure 1A, highlighting a possible constructive configuration thereof; and
▪ Figure 4 shows an exemplary diagram of a control system associated to a sampling apparatus incorporating the device of Figure 1A.

### Detailed description of preferred embodiments

Referring initially to Figures 1A and 1B, an auxiliary device suitable to allow exhalate collection on a sampling means, and in particular on an adsorbent cartridge, is generally denoted by 1.

The device 1 comprises a main body 2, having a longitudinal axis A. Preferably, the body 2 is elongated along said axis A and has a substantially cylindrical geometry.

In the present example, an inlet channel 200 for the expiratory flow and an outlet channel 210 for feeding the exhalate to an environment accommodating the adsorbent cartridge are manufactured integral with - or anyhow are made integral to - the main body 2. Such inlet 200 and outlet 210 channels open each, to the outside of the main body 2, into a respective inlet port 20 and outlet port 21.

The inlet port 20 is apt to be set in communication with a patient's mouth to receive just the expiratory flow. The outlet port 21, as mentioned above, is apt to be set in communication with the environment accommodating the adsorbent cartridge. Therefore, as it will be illustrated in greater detail hereinafter, in use the expiratory flow flows into the device 1 between the inlet port 20 and the outlet port 21.

Preferably, the outlet port 21 is arranged along the main body 2 in an intermediate position, so as to lie as far as possible from any perturbation of the flow of exhalate generated by flow interdicting/enabling means which will presently be disclosed.

The main body 2 further has, at its own longitudinal ends, a first and a second port for communication with the outside, respectively denoted by 22 and 23.

In the present example, the two inlet 20 and outlet 21 ports are associated to the side skirt of the main body 2, whereas the ports 22 and 23 for communication with the outside are made at opposite longitudinal ends thereof, preferably aligned or substantially aligned along the longitudinal axis A.

Inside the main body 2 a sampling chamber 101, having the outlet port 21, and an inhalation chamber 102, having the port 22 for communication with the outside, are obtained, the latter suitable to allow inletting of outside air into the inhalation chamber 102.

Preferably, the two chambers 101 and 102 are them also substantially aligned along the longitudinal axis A and arranged respectively the one downstream of the other one with respect to the flow of exhalate flowing between the inlet port 20 and the outlet port 21. Moreover, preferably both chambers 101 and 102 have a substantially cylindrical geometry.

At the portion of main body 2 interposed between inlet port 20 and outlet port 21, first passive-type flow interdicting/enabling means are arranged, denoted by 31. Such first means 31 is interposed between the chambers 101 and 102 and is apt to selectively set in flow communication the inhalation chamber 102 with the sampling chamber 101.

Likewise, second and third passive-type flow interdicting/enabling means are arranged at respectively the first 22 and second 23 port for communication with the outside, and denoted respectively by 32 and 33. Such means 32 and 33 are apt to selectively set in communication with the outside the respective ports 22 and 23. In particular, the second means 32 is suitable to allow a selective feeding of outside air into the inhalation chamber 102. The third means 33 is suitable to allow a selective outletting of exhalate to the outside.

The flow interdicting/enabling means 31, 32 and/or 33 typically consist in one or more check valves.

In a variant embodiment, the above-mentioned means 31, 32 and/or 33 may be suitable to allow an adjustment of the rate of flow therethrough, instead of having only the two above-mentioned supplying and interdicting configurations.

The overall configuration of the device 1 is such as to enable the operating modes described hereinafter.

As shown in Figure 1A, in the presence of a flow of exhalate being inlet from the port 20, the first means 31 opens to allow flow transit into the sampling chamber 101 and to the outlet port 21. At the same time, the second means 32 closes to prevent exhalate outletting to the outside of the inhalation chamber 102. Concomitantly, also the third means 33 opens to enable an outletting to the outside of the flow of exhalate transiting into the sampling chamber 101 without being captured at the outlet port 21. Feeding of the flow of exhalate through the outlet port 21 is obtained also by means of a pump or an equivalent means operating inside or in the proximity of the environment accommodating the adsorbent cartridge, with the purpose of guaranteeing a constant flow in the on-cartridge adsorbing operation.

As shown in Figure 1B, in case of negative pressure created by the inspiratory act of the patient, the operating modes become opposite to the hereto-described ones. In particular, the first means 31 closes to interdict any outflow from the sampling chamber 101 to the inlet port 20 and therefore to the patient's mouth. The second means 32 opens to allow outside air to enter the inhalation chamber 102 and just be inhaled through the inlet port 20. The third means 33 also closes, to prevent a further outletting of the residual flow of exhalate. Thus, a carrying on of the sampling of the exhalate collected in the sampling chamber 101 in the preceding expiratory step is allowed even during the inspiratory act.

Therefore, a continuous sampling of exhaled air is possible even during the inhalation step.

In particular, the inhalation chamber 102 as associated to the first and second flow interdicting/enabling means 31 and 32 substantially implements an assembly *"not re-breathing"* the flow of exhalate.

Therefore, by now it will be better appreciated that the described arrangement allows to separate the paths of air in the exhalating and inhalating steps, and to separate exhaled air in order to allow its sampling. Moreover, the fact that the above-mentioned chambers and the associated components are all manufactured at a single main body makes the device extremely compact.

It will be appreciated that the arrangement described, in particular in connection to the presence of the sampling chamber 101 and to the outlet port 21 in an intermediate position thereon, prevents exhaled air contained inside the chamber 101 to be entirely sampled before a new flow of exhalate is had. In that case, in fact, pumping into the cartridge environment air rather than exhaled air would be likely, with the risk of altering the measurement. Considering a flow rate of the above-mentioned sampling pump of about 80 ml/min, the dimensions of the chamber 101 may be calculated so as to obtain a volume of about 80 ml, which guarantees a correct measurement even in case, for some reason, the patient comes off the mouthpiece for a short time (up to about 30 s).

Air sampled through the device 1 is mainly alveolar air: the dead-space portion transits very quickly into the device and is the first one exiting through the port 23 for communication with the outside. The dead space is then sampled over a very short time for each expiratory act, therefore its influence on measurements is reduced. This is all to the advantage of the reproducibility of the sampling procedure, since dead-space air composition can be influenced by numerous confusing factors.

Moreover, it should be remarked that the device 1 performs the sampling on many expiratory acts, gradually integrating the information therefrom. In this case as well, it is an aspect apt to improve reproducibility.

Figure 2 shows an example of practical embodiment of the device 1, based on operating blocks assembled *ad hoc.* In particular:
- the first operating block A is associated to the inhalation chamber 102, and therefore bears the port 22 for communication with the outside and the first and second flow interdicting/enabling means 31 and 32, therefore implementing a block *"not re-breathing"* exhalate in the sense already illustrated;
- the second block B is associated to the inlet channel 200 and the related inlet port 20,
- the third block C is associated to the intermediate portion of the main body, i.e. substantially to the sampling chamber 101, bearing the outlet channel 210 with the related port 21; and
- the fourth block D is associated to the portion of the main body 2 distal to the patient and bearing the third interdicting/enabling means 33 and the related port 23 for communication with the outside.

The above-mentioned structural subdivision is further detailed in Figure 3, in which for simplicity's sake the connection with the adsorbent cartridge environment has not been depicted. From said figure it can be appreciated that the first and second interdicting/enabling means 31 and 32 are formed each by a pair of check valves arranged in parallel. Such valves may be of commercial type, preferably single-use ones (e.g. those produced by *Quin Tron Instrument Company*), therefore they will no further be dwelt upon.

With regard to the third means 33, it should ensure optimum sealing during a patient's inhaling, even though at this stage it is not subjected to any appreciable (passive) pressure gradient caused by the inspiratory flow, and this since the first means 31 is closed. A preferred embodiment of such third means 33 in the form of a check valve is shown just in Figure 3.

Such a valve has a configuration substantially shaped like a movable cap 331, apt to engage the distal longitudinal end of the main body 2, i.e. of the sampling chamber 101.

The cap 331 is apt to close the port 23 for communication with the outside by virtue of its weight, i.e. of the force of gravity.

Preferably, the cap-shaped body 331 is made of Delrin or of a material with equivalent mechanical properties.

At an internal surface of the cap 331 facing the side skirt of the main body 2, the cap 331 has a plurality of closure members 332, each in the form of an elongated projection, preferably cylindrical. Each of said members 332 is associated to a corresponding air outlet hole 232 of the main body 2, said holes overall defining the port 23 described above. In the present example six closure members 332 and six corresponding holes 232, preferably equally distributed, are provided.

During an expiratory flow, flow pressure allows lifting of the cap 331, with the entailed freeing of the holes 232 from the members 332, so that exhalate may flow to the outside. In the absence of said flow, and in particular during the inspiratory act, the cap 331 falls by gravity, in abutment on the main body 2, and the holes 232 are then obstructed by the members 332.

The pressure needed to lift the cap 331 can be equal to or lower than 1 cmH₂O, a very low value, such as not to be troublesome for patients suffering from respiratory illnesses.

It will be appreciated that the solution proposed hereto for the manufacturing of the means 33 is concomitantly effective and simple to make.

In use, the hereto-described device 1 may be part of an exhalate sampling apparatus, which comprises, besides the device 1, also the sampling means, as mentioned preferably an adsorbent cartridge, the latter arranged or apt to be arranged in an environment in communication with the outlet port 21.

Such apparatus typically comprises at least one mouthpiece apt to be placed in the patient's mouth to convey the exhalate thereof and to be connected to the inlet port 20, which mouthpiece is preferably single-use.

To the environment accommodating the adsorbent cartridge - or other sampling means - it is associated, as already mentioned, a pump - or an equivalent means - apt to produce a suction of the flow of exhalate at the outlet port 21. Figure 4 shows a block diagram of a control system, in particular a system for voltage regulation and timing, of said pump, denoted therein by 500.

The pump may be connected to the environment of the adsorbent cartridge and/or to the outlet port 21 by a preferably single-use tube for sucking inert material.

The control system shown in Figure 4 envisages, operatively connected the one to the other:
▪ a power supply block 501, typically a battery timer circuit;
▪ a voltage regulator block 502, able to provide a stabilized voltage, preferably from 1.2 V;
▪ a timing block 503, typically an integrated circuit;
▪ a switching block 504 for excluding the timing block 503 and therefore allowing manual operation of the pump;
▪ a first switch ("START") for starting the sampling procedure, which ends automatically after a preset time;
▪ a second switch ("RESET"), allowing to reset the timing.

The above-mentioned components are individually available on the market, therefore their description will no further be dwelt upon.

Both the supply voltage of the pump and the sampling time can be adjustable by so-called trimmers. Power supplying of the system can be obtained with four AA batteries, housed at the rear.

Finally, it is possible to imagine an integration of the above-mentioned device and/or apparatus in a more complex system, provided with other functionalities - e.g., spirometry or DLCO - so as to obtain a complete instrument for monitoring the respiratory function.

## Claims

1. An exhalate sampling apparatus, comprising an auxiliary device (1) for exhalate collection upon a sampling adsorbent cartridge, which devices comprises:
- a sampling chamber (101) having an outlet port (21), the latter arranged in a intermediate position on said sampling chamber (101) and apt to be put in communication with an environment which houses the sampling cartridge, said sampling chamber (101) having also a further port (23) for communication with the outside, arranged at said sampling chamber (101) downstream of said outlet port (21) with respect to a flow exhalate;
- an inhalation chamber (102) having a port (22) for communication with the outside, the latter apt to allow inletting of outside air into the inhalation chamber (102);
- first flow interdicting/enabling means (31) of a passive-type, arranged between said sampling (101) and inhalation (102) chambers and apt to selectively put in communication these latter chambers;
- second flow interdicting/enabling means (32) of a passive-type, arranged at said port (22) for communication with the outside of said inhalation chamber (102) and apt to selectively allow an inletting of outside air into said inhalation chamber (102),
- a single main body (2) inside which said inhalation (102) and sampling (101) chambers are contained, said chambers being arranged in sequence with respect to the flow of exhalate, the inhalation chamber (102) upstream of the sampling chamber (101), and substantially aligned along a longitudinal axis (A) of the device (1),
wherein said device (1) further has an inlet port (20) apt to be put in communication with a patient's mouth for receiving a flow of exhalate, which inlet port (20) is arranged at said inhalation chamber (102) and interposed between said port (22) for communication with the outside and said first flow interdicting/enabling means (31), wherein said inlet (20) port of said inhalation chamber (102) and said outlet port (21) of said sampling chamber (101) are associated with a side skirt of said main body (2) whereas said ports (22, 23) for communication with the outside are made at opposite longitudinal ends of the device (1),
wherein the overall configuration is such that, during an expiratory act causing a flow of exhalate being inlet from said inlet port (20), said first means (31) puts in communication said inhalation chamber (102) with said sampling chamber (101) and said second means (32) interdicts the inletting of outside air into said inhalation chamber (102), and vice versa during an inspiratory act, and wherein said sampling apparatus comprises said adsorbent cartridge as sampling means which cartridge is arranged in the environment in communication with said outlet port (21),
**characterized in that** said sampling apparatus futher comprises third flow interdicting/enabling means (33), of a passive-type, arranged at said further port (23) for communication with the outside and apt to selectively set said sampling chamber (101) in communication with the outside in the presence of an expiratory act,

2. The sampling apparatus according to claim 1, wherein said single main body (2) has a substantially cylindrical configuration.

3. The sampling apparatus according to any one of the preceding claims, wherein said third flow interdicting/enabling means (33) comprises a cap-shaped body (331) apt to engage by gravity said further port (23) for communication with the outside.

4. The sampling apparatus according to to any one of the preceding claims, wherein said further port (23) for communication with the outside is made in the form of one or more holes (232) and said third flow interdicting/enabling means (33) has one or more corresponding closure members (332) of such one or more holes (232).

5. The sampling apparatus according to any one of the preceding claims, wherein said first (31), second (32) and/or third (33) flow interdicting/enabling means comprise one or more check valves.

6. The sampling apparatus according to any one of the preceding claims, wherein said first (31), second (32) and/or third (33) flow interdicting/enabling means are configured to allow an adjustment of the rate of the flow.

7. The apparatus according to the preceding claim, comprising at least one mouthpiece apt to be placed in the patient's mouth to convey the exhalate thereof and to be connected to said inlet port (20), which mouthpiece is preferably single-use.

## Patentansprüche

1. Vorrichtung zur Probennahme von Ausatemluft, die umfasst: eine Hilfsvorrichtung (1) zur Sammlung von Ausatemluft auf einer absorbierenden Probennahmekartusche, wobei die Vorrichtungen umfassen:
- eine Probennahmekammer (101), die über eine Auslassöffnung (21) verfügt, wobei Letztere bei einer Zwischenstellung auf der Probennahmekammer (101) angeordnet ist und geeignet ist in Kommunikation mit einer Atmosphäre gebracht zu werden, die die Probennahmekartusche ausfüllt, wobei die Probennahmekammer (101) außerdem über eine weitere Öffnung (23) zur Kommunikation mit der Außenumgebung verfügt, die bei der Probennahmekammer (101), bezüglich eines Ausatemluftstroms, flussabwärts von der Auslassöffnung (21) angeordnet ist;
- eine Inhalationskammer (102), die über eine Öffnung (22) zur Kommunikation mit der Außenumgebung verfügt, wobei die Letztere geeignet ist einen Einlass von Außenluft in die Inhalationskammer (102) zu erlauben;
- ein erstes Strömungsverhinderungs-/freigabemittel (31) passiver Art, das zwischen der Probennahmekammer (101) und der Inhalationskammer (102) angeordnet ist und geeignet ist diese letzteren Kammern selektiv in Kommunikation zu bringen;
- ein zweites Strömungsverhinderungs-/freigabemittel (32) passiver Art, das bei der Öffnung (22) zur Kommunikation mit der Außenumgebung der Inhalationskammer (102) angeordnet ist und geeignet ist einen Einlass von Außenluft in die Inhalationskammer (102) selektiv zu erlauben;
- einen Hauptkorpus (2), in dem die Inhalationskammer (102) und die Probennahmekammer (101) untergebracht sind, wobei die Kammern bezüglich des Ausatemluftstroms in Reihe geschaltet sind, wobei die Inhalationskammer (102) stromaufwärts von der Probennahmekammer (101) angeordnet und im Wesentlichen entlang einer Längsachse (A) der Vorrichtung (1) ausgerichtet ist,
wobei die Vorrichtung (1) weiterhin umfasst: eine Einlassöffnung (20), die geeignet ist zum Empfang eines Ausatemluftstroms in Kommunikation mit dem Mund eines Patienten gebracht zu werden, wobei die Einlassöffnung (20) bei der Inhalationskammer (102) zwischen der Öffnung (22) zur Kommunikation mit der Außenumgebung und dem ersten Strömungsverhinderungs-/freigabemittel (31) angeordnet ist, wobei die Einlassöffnung (20) der Inhalationskammer (102) und die Auslassöffnung (21) der Probennahmekammer (101) mit einem Seitenschweller des Hauptkorpus (2) verknüpft sind, wobei die Öffnungen (22, 23) zur Kommunikation mit der Außenumgebung an in Längsrichtung gegenüberliegenden Enden der Vorrichtung (1) dargestellt werden,
wobei die Gesamtkonfiguration so ist, dass, während einer exspiratorischen Aktion, die verursacht, dass ein Ausatemluftstrom von der Einlassöffnung (20) eingelassen wird, das erste Mittel (31) die Inhalationskammer (102) mit der Probennahmekammer (101) in Kommunikation bringt und das zweite Mittel (32) den Einlass von Außenluft in die Inhalationskammer (102) verhindert und, während einer inspiratorischen Aktion, umgekehrt und wobei die Probennahmevorrichtung die absorbierende Kartusche als Probennahmemittel umfasst, wobei die Kartusche so angeordnet ist, dass die Atmosphäre in Kommunikation mit der Auslassöffnung (21) ist,
**dadurch gekennzeichnet, dass** die Probennahmevorrichtung weiterhin umfasst: ein drittes Strömungsverhinderungs-/freigabemittel (33) passiver Art, das bei der weiteren Öffnung (23) zur Kommunikation mit der Außenumgebung angeordnet ist und geeignet ist bei Vorhandensein einer exspiratorischen Aktion die Probennahmekammer (101) selektiv in Kommunikation mit der Außenumgebung zu bringen.

2. Probennahmevorrichtung gemäß Anspruch 1, wobei der einzelne Hauptkorpus (2) über eine im Wesentlichen zylindrische Konfiguration verfügt.

3. Probennahmevorrichtung gemäß einem der vorangehenden Ansprüche, wobei das dritte Strömungsverhinderungs-/freigabemittel (33) umfasst: einen deckelförmigen Korpus (331), der geeignet ist die weitere Öffnung (23) zur Kommunikation mit der Außenumgebung durch Schwerkraft einzusetzen.

4. Probennahmevorrichtung gemäß einem der vorangehenden Ansprüche, wobei die weitere Öffnung (23) zur Kommunikation mit der Außenumgebung in der Form eines oder mehrerer Löcher (232) dargestellt wird und das dritte Strömungsverhinderungs-/freigabemittel (33) über ein oder mehrere entsprechende Verschlussteile (332) eines oder mehrerer solcher Löcher (232) verfügt.

5. Probennahmevorrichtung gemäß einem der vorangehenden Ansprüche, wobei das erste (31), zweite (32) und/oder dritte (33) Strömungsverhinderungs-/freigabemittel ein oder mehrere Rückschlagventile umfassen.

6. Probennahmevorrichtung gemäß einem der vorangehenden Ansprüche, wobei das erste (31), zweite (32) und/oder dritte (33) Strömungsverhinderungs-/freigabemittel geeignet sind eine Justierung der Strömungsrate zu erlauben.

7. Vorrichtung gemäß dem vorangehenden Anspruch, die umfasst: mindestens ein Mundstück, das geeignet ist in den Mund eines Patienten gesteckt zu werden, um die Ausatemluft davon zu übertragen, und mit der Einlassöffnung (20) verbunden zu sein, wobei das Mundstück vorzugsweise ein Einmal-Mundstück ist.

## Revendications

1. Appareil d'échantillonnage d'expiration, comprenant un dispositif auxiliaire (1) pour la collecte de l'expiration sur une cartouche adsorbante d'échantillonnage, lequel dispositif comprend :
- une chambre d'échantillonnage (101) ayant un orifice de sortie (21), ce dernier étant agencé dans une position intermédiaire sur ladite chambre d'échantillonnage (101) et apte à être placé en communication avec un environnement qui loge la cartouche d'échantillonnage, ladite chambre d'échantillonnage (101) ayant également un autre orifice (23) pour la communication avec l'extérieur, agencé sur ladite chambre d'échantillonnage (101) en aval dudit orifice de sortie (21) par rapport à une expiration de flux ;
- une chambre d'inspiration (102) ayant un orifice (22) pour la communication avec l'extérieur, ce dernier étant apte à permettre l'entrée de l'air extérieur dans la chambre d'inspiration (102) ;
- un premier moyen d'interdiction/d'autorisation de flux (31) d'un type passif, agencé entre lesdites chambres d'échantillonnage (101) et d'inspiration (102) et apte à placer sélectivement ces deux dernières chambres en communication ;
- un deuxième moyen d'interdiction/d'autorisation de flux (32) d'un type passif, agencé au niveau dudit orifice (22) pour la communication avec l'extérieur de ladite chambre d'inspiration (102) et apte à permettre sélectivement une entrée de l'air extérieur dans ladite chambre d'inspiration (102),
- un corps principal (2) unique à l'intérieur duquel sont contenues lesdites chambres d'inspiration (102) et d'échantillonnage (101), lesdites chambres étant agencées en séquence par rapport au flux d'expiration, la chambre d'inspiration (102) étant en amont de la chambre d'échantillonnage (101) et sensiblement alignée le long d'un axe longitudinal (A) du dispositif (1),
dans lequel ledit dispositif (1) a en outre un orifice d'entrée (20) apte à être placé en communication avec la bouche d'un patient pour recevoir un flux d'expiration, ledit orifice d'entrée (20) est agencé au niveau de ladite chambre d'inspiration (102) et intercalé entre ledit orifice (22) pour la communication avec l'extérieur et ledit premier moyen d'interdiction/d'autorisation de flux (31), dans lequel ledit orifice d'entrée (20) de ladite chambre d'inspiration (102) et ledit orifice de sortie (21) de ladite chambre d'échantillonnage (101) sont associés à une jupe latérale dudit corps principal (2) alors que lesdits orifices (22, 23) pour la communication avec l'extérieur sont réalisés au niveau des extrémités longitudinales opposées du dispositif (1),
dans lequel la configuration globale est telle que pendant un acte expiratoire amenant un flux d'expiration à entrer par ledit orifice d'entrée (20), ledit premier moyen (31) met en communication ladite chambre d'inspiration (102) avec ladite chambre d'échantillonnage (101) et ledit deuxième moyen (32) interdit l'entrée de l'air extérieur dans ladite chambre d'inspiration (102) et vice versa pendant un acte d'inspiration,
et dans lequel ledit appareil d'échantillonnage comprend ladite cartouche adsorbante en tant que moyen d'échantillonnage, laquelle cartouche est agencée dans l'environnement en communication avec ledit orifice de sortie (21),
**caractérisé en ce que** ledit appareil d'échantillonnage comprend en outre un troisième moyen d'interdiction/d'autorisation de flux (33) d'un type passif, agencé au niveau dudit orifice (23) supplémentaire pour la communication avec l'extérieur et apte à placer sélectivement ladite chambre d'échantillonnage (101) en communication avec l'extérieur en présence d'un acte expiratoire.

2. Appareil d'échantillonnage selon la revendication 1, dans lequel ledit corps principal (2) unique a une configuration sensiblement cylindrique.

3. Appareil d'échantillonnage selon l'une quelconque des revendications précédentes, dans lequel ledit troisième moyen, d'interdiction/d'autorisation de flux (33) comprend un corps en forme de capuchon (331) apte à mettre en prise, par gravité, ledit orifice (23) supplémentaire pour la communication avec l'extérieur.

4. Appareil d'échantillonnage selon l'une quelconque des revendications précédentes, dans lequel ledit orifice (23) supplémentaire pour la communication avec l'extérieur est réalisé sous la forme d'un ou de plusieurs trous (232) et ledit troisième moyen d'interdiction/d'autorisation de flux (33) a un ou plusieurs éléments de fermeture (332) correspondants de ces un ou plusieurs trous (232).

5. Appareil d'échantillonnage selon l'une quelconque des revendications précédentes, dans lequel lesdits premier (31), deuxième (32) et/ou troisième (33) moyens d'interdiction/d'autorisation de flux comprennent un ou plusieurs clapets anti-retour.

6. Appareil d'échantillonnage selon l'une quelconque des revendications précédentes, dans lequel lesdits premier (31), deuxième (32) et/ou troisième (33) moyens d'interdiction/d'autorisation de flux sont configurés pour permettre un ajustement du débit.

7. Appareil selon la revendication précédente, comprenant au moins un embout buccal apte à être placé dans la bouche du patient pour transporter son expiration et à être raccordé audit orifice d'entrée (20), lequel embout buccal est de préférence à usage unique.
